(19) **Europäisches Patentamt**
**European Patent Office**
**Office européen des brevets**

(11) **EP 2 692 289 A1**

(12) **EUROPEAN PATENT APPLICATION**

(43) Date of publication:
**05.02.2014 Bulletin 2014/06**

(51) Int Cl.:
*A61B 5/00* (2006.01)        *A61B 5/05* (2006.01)
*A61B 8/08* (2006.01)

(21) Application number: **13178301.1**

(22) Date of filing: **29.07.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **29.07.2012 US 201213561047**
**29.07.2012 US 201213561048**

(71) Applicant: **Ultrawave Labs Inc.**
**Newport Beach, CA 92660 (US)**

(72) Inventors:
• **Ismail, Aly M.**
**Newport Beach, CA California 92660 (US)**
• **Carr, Francis**
**Newport Beach, CA California 92660 (US)**
• **Kwok, Sai**
**Newport Beach, CA California 92660 (US)**

(74) Representative: **Bast, Tim**
**Zugerstrasse 80a**
**6318 Walchwil (CH)**

(54) **Multi-modality ultrasound and radio frequency system for imaging tissue**

(57)     This invention providesfor performing characterization and imaging of tissue, tumors, structures, lesions, and ablations under investigation. Specifically, the invention couples ultrasound technology comprising at least two focused ultrasound beams for vibrating target tissues located at the focal point of the ultrasound beams intersection with a radio frequency system for measuring the response of the target tissues. The ultrasound system vibrates the target tissues while the reflected radio frequency energy is transmitted into the target tissues. When reflected, the main carrier tone of the reflected radio frequency energy is cancelled and analysis is performed on the remaining sideband frequencies.

This invention provides a dual-modality system for imaging target tissue, e.g. for the detection of cancerous tissues. Ultrasound technology comprising at least two focused ultrasound beams (108, 110) for vibrating target tissues located at the focal point (112) of the ultrasound beams intersection is coupled with a radio frequency (RF) system (102) for measuring the response of the target tissues. The ultrasound system vibrates the target tissues while the reflected radio frequency energy is transmitted into the target tissues. A cancellation module (138) is provided to eliminate the main carrier tone of the reflected RF signal as well as signal leakage from the transmit antenna (130), leaving only the sidebands that are generated due to the Doppler shift resulting from the vibration of the target tissues when subjected to the focused ultrasound waves.

**Fig. 1**

EP 2 692 289 A1

**Description**

**BACKGROUND OF THE INVENTION**

**1. Field of Invention.**

The field of the invention is soft tissue imaging for deep tissue and tissues within the body using radio frequency energy and vibro-acoustography in a dual modality imaging system where the two modalities operate simultaneously.

**2. Related Art.**

A number of imaging technologies are available to detect cancer in soft and compressed tissues within the human body. For breast cancer, X-ray mammography is currently the only FDA approved technology for breast cancer screening. However, mammography results in too many false negatives and false positives which delays the detection of early cancer, or results in unnecessary biopsies and expense. Additionally, mammography results are unreliable in the case of dense or fibrocystic breast tissues. This makes mammography unsuitable for cancer detection in young women with familial history and dense tissues and it further exposes these women to the danger of frequent exposure to ionizing radiation. Magnetic Resonance Imaging (MRI) achieves much more reliable cancer detection results within the body and for breast cancer. However, the cost of MRI is prohibitive and the procedure is too long and results in discomfort and anxiety for many patients. Due to its lower cost and ease of application, Ultrasound is currently being used for further diagnosis of subjects with abnormal mammograms. An ultrasound imaging system uses a transducer to transmit high frequency ultrasound pressure waves into the target tissue and measures the reflected waves, which in turn are converted into electric signal to display the image. The reflected signal depends on the elasticity modulus of the tissue under investigation which may vary from 1 to 10% between cancerous and non-cancerous tissues. While ultrasound images achieve an excellent resolution and reasonable depth of penetration, the resulting images have relatively poor contrast.

Over the past decade, research has turned to microwave radar techniques for soft tissue imaging. Microwave detection relies on the difference of permittivity between cancerous and benign tissues due to the difference in water content between the two types of tissues. This offers significant improvement in contrast and detection and diagnostic capacity over ultrasound and other anatomical imaging modalities. However, since the wavelength of microwaves in soft tissue is on order of a few centimeters, the spatial resolution of this method is limited. As a result, imaging methods that rely on microwaves alone are disadvantaged by the necessity to trade off low-frequency penetration against high-frequency contrast.

To meet the challenges of cancer detection in deep soft tissues, it is desired to achieve high penetration, high resolution, fast scanning and high contrast. This proved to be quite challenging using any single imaging modality because, used alone, each has significant limitations. To circumvent these limitations recent efforts have focused on aggregating information from different modalities to achieve a better imaging system. This can be done by either combining the resultant data or images from each modality or by combining the modalities themselves in a single imaging system.

The Rosner et al. reference U.S. Patent Application No. 2007/0276240 A1 discloses a system which uses both acoustic and microwave methods for imaging. The Rosner *et al.* reference uses microwave excitation and detection independently of ultrasound excitation and detection in order to generate images of particular locations in the target media. First, the Rosner *et al.* reference uses the ultrasound array source to transmit acoustic energy into the target medium. In response to the stimulus, an ultrasound transducer receives an echo from the target medium and generates electrical signals representing the ultrasound image. Then he uses a microwave source sequentially to transmit a microwave signal to the target tissues. A microwave transducer receives an echo from the target in response to microwave stimulus and generates a second electrical signal representing the microwave image of the target tissues. The two independent electrical signals resulting from the microwave and the ultrasound transducers are summed into a processing unit and are used in part or in full to generate one or more images of the target tissue that beneficially combine the information contained in the two independently generated images. However, in order to generate an image of reasonable resolution the energy must be tightly focused inside the target medium. It is very difficult to obtain a small spot size with a focused beam of microwave energy even if an array is used. To obtain the smallest feasible spot size one needs to employ a very large array. Such large arrays would be too large and impractical to address parts of human or animal anatomy. Additionally, to make the spot size small, the energy from every one of the antenna apertures must arrive at the focal point simultaneously. For short range propagation, as would be the case with any part of human or animal anatomy, the beam propagation times from the common source to the target tissue need to be matched with picosecond-level precision so that the beams all arrive with peak energy simultaneously. Such precision timing requires a very complex computer system and extensive microwave and digital hardware to implement. Regardless of the implementation chosen, the minimum focal spot size for the microwave beam would be on the order of one (1) centimeter due to diffraction limitations and internal scattering resulting for the inherent inhomogeneity of the human body.

This simple integration using independent electrical signals from the ultrasound and microwave modalities, as proposed by the Rosner *et al.* reference, does not take advantage of the physical interaction of the ultrasound and microwave modalities. Therefore, this method of combining the image results from microwave and from ultrasound possesses the same disadvantages of each subsystem when used independently. It is obvious to those skilled in the art that the proposed system fails to concurrently achieve the desired combination of high penetration, high resolution, and high contrast. Additionally, in the case of the Rosner *et al.* reference, as a result of the microwave wavelength, the detection is the result of large area excitation which inherently results in low resolution of microwaves which make the Rosner *et al.* reference's teaching capable of only detecting targets with centimeter dimensions. Also, the proposed teaching fails to address the problem inherent in short range radar system where it is impossible to turn off the transmitter during the reflected signal detection causing tremendous degradation to the signal-to-noise ratio and the overall imaging scheme.

Rather than the independent use of two modalities for generating images, the Parker et al. (U.S. Patent No. 5,099,848) reference teaches the use of broad area, acoustic beams that sweep in frequency from approximately 1 to 1000 Hz. This unfocused, swept frequency beam generates acoustic resonances at frequencies determined by the size and shape of a predetermined geometry such as a cylinder that surrounds the portion of the human or animal anatomy that will be examined. Hence the Parker reference's teaching is only applicable to parts of the human anatomy that can be surrounded by a geometrical structure. Conversely, other parts of human or animal anatomy that lack significant protrusion from the body could not be imaged.

According to the Parker reference's teachings the image generated is based on the difference in elastic contrast between cancerous and non-cancerous tissue. It is well known to those skilled in the art that the magnitude of the difference in elastic constants for these two types of tissue is relatively small, typically on the order of 1 to 10% percent, thus resulting in a much poorer contrast compared to methods that use the difference in dielectric contrast which, as pointed out by Li *et al.* is on the order of 400%. According to the Parker reference's teachings acoustic resonances of anatomically loaded predetermined structure will generate acoustic standing waves within the target tissues constrained by that structure. However, there is no *a priori* reason that the standing waves will induce significant tissue motion at the precise tumor location. The standing wave patterns are determined by the dimensions of the surrounding mechanically rigid shape and the acoustic loading by the anatomy confined by the mechanically rigid shape. In fact, it is possible that a particular acoustic mode produces no motion at a tumor location due to the existence of an acoustic null at the tumor location. The Parker *et al.* reference attempts to address this issue by sweeping the frequency of the acoustic energy over board range and examining the frequency shift of multiple modes. Such an approach will be feasible only if the resonant frequencies are not too closely spaced. The uncertainty of this approach is highlighted by the Parker *et al.* reference as he indicates the desirability of having a model for comparison with measured results. Using the opposite breast for comparisons in the case of breast cancer evaluations inherently assumes that one breast can provide a pristine reference for the other breast, which is invalid assumption due to the difference in size, shape and homogeneity between the left and right breasts.

The acoustic losses of the breast-loaded cylinder will broaden the acoustic resonance, thus decreasing the amplitude of the standing waves and making precise determination of the frequency shift difficult. To reduce the difficulty in precisely locating a tumor, the Parker *et al.* reference uses a focused ultrasonic beam to determine the motion induced by the broad area acoustic wave. This ultrasonic readout is shifted in frequency due to the motion induced by the low frequency, broad-area acoustic wave. The contrast of this all acoustic-based system would be expected to be relatively modest due to the small difference in elastic constants between cancerous and non-cancerous tissue.

The Parker *et al.* reference further suggests that microwave energy is an alternative to ultrasonic readout of the motion; however, microwave readout of motion requires that the microwave energy be coupled into the predetermined mechanically rigid shape effectively. Frequencies high enough to be unaffected by the predetermined mechanically rigid shape as might be the case for millimeter wave radiation are highly absorbed by the surrounding tissues and thus cannot be used. For frequencies in the lower part of the microwave band near 1GHz where absorption is not a limitation, the focusing capability of the microwave energy would be limited to spots somewhat larger than one centimeter. Further, coupling the microwave energy into the predetermined mechanically rigid shape could be difficult if the predetermined mechanically rigid shape is anywhere near an electromagnetic resonant frequency. Yet good coupling to a precise location is necessary to clearly identify tumor location and its properties.

If microwaves were used for readout in the configuration as taught by the Parker *et al.* reference, there would inevitably be significant reflection from multiple interfaces that could partially reflect the microwave energy into a receiving antenna in addition to possible the reflections into the receiving antenna from the surrounding predetermined mechanically rigid shape. The signal level of these spurious reflections is likely to be much higher in magnitude than the reflection due to the small difference in elastic properties the Parker *et al.* reference's teaching attempts to detect. The Parker *et al.* reference makes no mention of providing means for detecting the very small signal resulting from the inclusion in the presence of a large interfering signal. The small low frequency signal that the Parker *et al.* teaching requires to be extracted may be overwhelmed by the 1/f noise of the microwave oscillator that provides the microwave signal. Such noise is inherent in all oscillators and cannot be arbitrarily reduced or eliminated. In addition to the loss of contrast mentioned earlier, these factors may place further limits on the minimum size of tumors that may be detected with the Parker *et al.* reference methodology.

Meaney *et al.* reference U.S. Patent No. 6,448,788 teaches a method of determining the dielectric properties of an inhomogeneous medium by means of external measurement using multiple antennas disposed around the periphery of the object to be analyzed. According to the Meaney *et al.* reference's teaching the multiple antennas are immersed in a homogenous liquid solution such as a saline solution. Measurements are taken pairwise with the remaining, non-active antennas terminated to avoid unwanted reflections. The resulting data is used to estimate the electrical properties of the medium and the electric field within the medium. In order to estimate the electrical properties and electric field, the Meaney *et al.* reference teaches the use of an adaptive gridding technique which is well known in the field of computational electromagnetics. Despite considerable research in academia such as those expressed in the Li *et al.* reference and the reference "A Sparsity Regularization Approach to the Electromagnetic Inverse Scattering Problem" by David W. Winters, et al. (January 2010), means for precisely and expeditiously solving the inverse problem where internal properties of an inhomogeneous medium determined by means of external measurements in conjunction with computationally-based estimation of the internal fields and properties of the medium has proved to be prone to error. Additionally, the Meaney *et al.* reference because of its reliance on microwave only, suffers from the need to trade off low-frequency to achieve penetration against high-frequency to achieve high resolution.

The Dines *et al.* reference U.S. Patent No. 6,876,879 aggregates the use of X-ray mammography and high frequency ultrasound in spatial registration to generate a three dimensional image of compressed breast. The beast tissue is compressed between either two paddles in the case of ultrasound or between a top paddle and an X-ray detector in order to immobilize the breast and achieve the spatial registration. The hardware is configured to either allow the ultrasound probe or the X-ray head to image the compressed breast. While using spatial registration, the X-ray and the ultrasound images are done independently, the data from the X-ray image and the ultrasound images are fed into a processing unit where they are combined to generate a three dimensional image with the goal of enhancing the potential for early breast cancer detection. While the benefits and shortcomings of X-ray and ultrasound used singly for breast cancer detection are well known, the added value resulting from the Dines *et al.* reference's teaching on combining the images via digital processing or compressing the breast during ultrasound imaging is not clear. While there may some marginal benefit from the teachings of two overlapping images or creation of a three dimensional image, the Dines et al. methodology has disadvantages inherent in each of the two methods applied separately.

Another approach for combining modalities for breast cancer detection in soft tissues was proposed by the Li et al. reference in U.S. Patent No. 7,266,407. The Li *et al.* reference combines the use of microwaves and ultrasound waves by using microwaves to heat the tumor location thereby inducing thermoacoustic waves in the surrounding tissues while using ultrasound waves to detect the reflected acoustic signals. The tissue is radiated with a microwave pulse in the range of 1 to 10 GHz which is swept over a frequency range of about 1 GHz. This frequency range is selected to achieve sufficient penetration in human breast tissue. The microwave energy absorption causes the tissues to expand and radiate thermoacoustic waves in all directions. In the 1 to 10 GHz frequency range, the microwave absorption in tissues is a function of the tissue local water content which results in different permittivity and microwave energy absorption. Assuming breast tissue to be a homogeneous acoustic medium, the reflected acoustic signal carries information about the microwave absorption pattern in the breast tissue which in turn can be used to construct the breast image. The induced ultrasound signal is proportional to the temperature increase in the tumor which is a function of the absorbed microwave energy.

The Li *et al.* reference's approach has a number of shortcomings. Among these shortcomings are the inability to find a medium to achieve good ultrasound and microwave impedance matching into the breast tissue while uniformly heating the breast tissue due the variability in the breast size, shape and presence of inhomogeneity in the breast tissue. The skin layer covering the breast tissue presents another major challenge due the reflection of energy at the skin and the tank fluid and the skin and the breast tissue boundaries. This introduces significant signal clutter and interferes with the propagation path of thermoacoustic waves resulting in errors when performing the inverse calculation thus negatively impacting the image quality. The thermoacoustic waves will also be scattered by the inhomgeneities of the breast. The Li *et al.* reference proposes heating the breast tissue in segments and applying a variety of signal processing techniques to overcome these issues when doing the inverse calculation. However, none of the methods proposed can get around (1) the loss of contrast due to inhomogeneities obscuring the tumors located behind them, (2) shadowing by an inhomogeneity located in front of a tumor, or (3) determining ways to overcome the effect of attenuation as the microwave propagates through the breast tissue or to precisely account for the time of flight of the reflected thermoacoustic signals. These challenges become even greater when imaging a cystic or dense tissue.

In US Patent Application No. 2009/0281422 A1, Salama et al. suggest to use a multi-modality system for performing imaging of materials and objects in dense compressive media. An ultrasound subsystem is employed to excite a region in the dense compressive media and a microwave subsystem is employed to collect detection, characterization, and imaging information from the excited region. The resultant frequency and power date is transmitted to a display for viewing by a technician.

The main shortcoming of the system described by Salama *et al.* is the relatively poor signal to noise ratio.

Hence, a need exists for a multi-modality imaging system that overcomes the shortcomings of the prior art. A need exists for an imaging system that is very sensitive to the variations in the permittivity between benign and cancerous tissues, which may vary by a factor of five, while ultrasound sensitivity to these variations is less than 10%. Ultrasound methods rely on the measurement of variations in the mechanical properties of benign tissue and cancerous tumors, which are not large. On the other hand, microwave methods take advantage of the difference in dielectric constants associated with the water content of benign tissue and cancerous tumors, which vary dramatically. A need exists for a system that combines the superior penetration and resolution characteristics of focused high-frequency ultrasound input waves, with the superior penetration and contrast capacity of microwave detection and imaging.

## SUMMARY

This invention provides a real-time, simultaneously operating dual-modality system for performing characterization and imaging of tissue, tumors, structures, lesions, ablations and other abnormalities in tissues under investigation. Specifically, the invention couples ultrasound technology comprising at least two focused ultrasound beams for vibrating target tissues located at the focal point of the ultrasound beams intersection with a radio frequency ("RF") system for measuring the vibration-induced or other acoustically-induced changes of the target tissues. The ultrasound system vibrates the target tissues while the reflected radio frequency signals are used to image the vibrating tissues.

The use of two focused ultrasound beams with their focal points intersecting at a target inclusion buried in a larger mass of tissue provides the means to image submillimeter-size inclusions deep inside the tissues or inside the body. The ultrasound energy through its interaction with the nonlinearity of the inclusion induces low frequency vibration in the target inclusions which results in measurable displacement of the inclusion and causes a Doppler shift in the reflected radio frequency signals. The dielectric properties of the inclusion provide the means for contrast of the inclusion with respect to its surroundings and provide a superior contrast compared to other methods resulting in an approximately 30 to 40 dB signal-to-noise improvement. While vibro-acoustography used alone as an imaging modality takes advantage of low-frequency ultrasound harmonic components from multiple high-frequency ultrasound wave inputs to excite the target tissue, it relies on differences in elastic properties of tissue structures such as tumors, lesions, ablations, etc. relative to the surrounding tissue as the means to contrast the structure with its surroundings. Likewise microwave-only imaging modalities require solving the inverse problem which can produce imprecise results and is limited in resolution to a fraction of the wavelength used for measurement. This invention combines the superior penetration and resolution capabilities of ultrasound with the high contrast and discrimination of microwave imaging.

In this invention it should be noted that while the radio frequency energy and the ultrasound signals are applied concurrently on the target tissues in true dual modality system where both modalities work simultaneously, the ultrasound waves are used to vibrate the target tissues while the radio frequency energy provides the detection and imaging functionality. In an alternative embodiment, ultrasound transceivers can be used so that images can be generated by the radio frequency energy as well as the ultrasound waves. The vibration of the target tissue at the intersection of the two ultrasound beams results in Doppler sidebands around the radio frequency carrier frequency and separated from the carrier by a frequency equal to the difference in frequency of the two ultrasound signals. The ability to focus the ultrasound beams at points of intersection at a desired tissue depth dramatically improves the dynamic range of this imaging scheme and enables detecting and imaging sub-millimeter size targets and achieves high levels of resolution at a desired tissue depth while also achieving superior contrast.

Reflected radio frequency energy is received by a radio frequency antenna and subsequently analyzed. The main reflected tone may also comprise signal leakage and other non-target related reflected energy which is cancelled by a cancellation subsystem that uses the short propagation distances inherent in this system cancel the main tone to a predetermined set of criteria. The processing unit is used to analyze the reflected sideband frequencies so that target images can be generated. The focused ultrasound source is used to excite the target tissues causing them to vibrate while the radio frequency energy is used for the imaging by detecting the reflected energy and using the difference in dielectric properties (e.g., dielectric constant and loss factors) between normal and cancerous tissues due, for example, to the difference in the water content or the difference in reflectivity between ablated and normal tissues to provide discrimination.

One aspect of the invention is the use of the dual-modality system of radio frequency energy and ultrasound for direct imaging of deep soft tissues when investigating breast tissues or other tissues within the body cavity such as liver, pancreas or the heart tissues. The methodologies include a radio frequency transceiver that irradiates the targets with radio frequency energy at a specific frequency and measures the reflected signal, a focused ultrasound sound source to impart an acoustic force at a specific point and depth within the tissue and cause a vibratory motion or other changes in the dielectric properties of the tissue at a specific frequency at the specific point where the ultrasound energy impacts the target, this in turn results in the appearance of sidebands in the reflected radio frequency signals. The relative amplitude of the sidebands is a function of the dielectric properties of the tissues at the point of the ultrasound energy excitation. The reflected radio frequency energy is fed into a detector and signal processor that drives the image display.

The invention uses vibro-acoustography to induce the vibration at the desired target point within the tissue by applying two focused ultrasound beams which have a small difference in frequency to the target causing the tissue at the point of intersection of the two beams to vibrate at a frequency equal to difference between the two ultrasound frequencies which in turn results in sidebands equally spaced around the radio frequency carrier signal and separated from the carrier signal by the difference in frequency of the two ultrasound beams.

This invention may also be used to overcome the problems resulting from the inability to turn the microwave transmitter off during the reception of the reflected microwave signal by the receiver due to closeness of the target through the use of a cancellation method to eliminate the carrier or main tone thereby overcoming the unavoidable coupling between the transmit and receive antennas and dramatically boosting the signal-to-noise ratio and facilitating the extraction of the sideband signals.

Another aspect of this invention is the use of a method for scanning the target object to provide a two-dimensional image and provide means to construct a three dimensional image by the controlling the depth of focus of the Ultrasound beams or through the use of ultrasound transmitter arrays.

**Brief Description of the Drawings**

The components in the figures are not necessarily to scale, emphasis being placed instead upon illustrating the principles of the invention. In the figures, like reference numerals designate corresponding parts throughout the different views.

Figure 1 is block diagram illustrating the dual modality radio frequency / ultrasound system with the ultrasound subsystem and the radio frequency ("RF") subsystem.

Figure 2 is a block diagram illustrating two ultrasound transducers and the radio frequency antennas transmitting the ultrasound and radio frequency energy respectively in the target tissue while vibrating the target.

Figure 3 is a graph illustrating the reflected radio frequency energy when the vibro-acoustography modality is not implemented.

Figure 4 is a graph illustrating the reflected radio frequency energy with the associated sidebands when the radio frequency energy is used in the presence of vibro-acoustography modality.

Figure 5 is a schematic illustrating a block diagram of the radio frequency subsystem as used with the ultrasound transmitters where the two radio frequency antennas transmit the radio frequency energy into the target tissue while the ultrasound energy vibrates the target tissue.

Figure 6 is a flow chart of the methodology of the dual modality imaging system performing a scan of target tissues.

Figure 7 is a flow chart of the methodology of the scanning functionality of the imaging system.

**DETAILED DESCRIPTION**

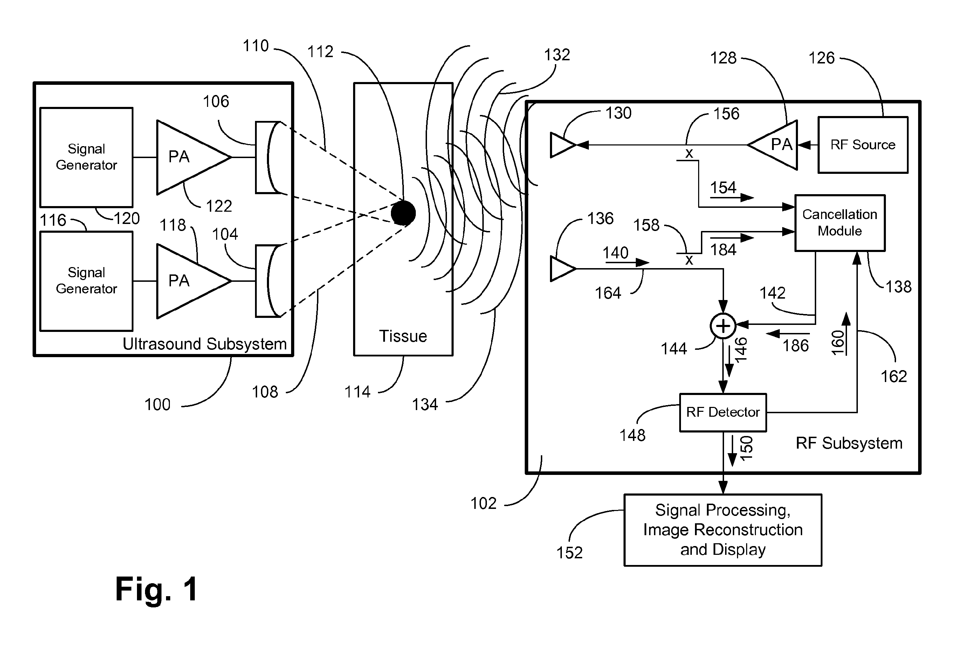

Figure 1 is a block diagram illustrating the dual-modality imaging system where the two modalities are operating at the same time. The system comprises an ultrasound subsystem 100 and a radio frequency subsystem 102. The ultrasound subsystem 100 employs at least two ultrasound transmitter 104 and 106 capable of generating two focused ultrasound beams 108 and 110 aimed at a focal point 112 where the intersecting ultrasonic beams are focused within the tissue 114 under observation. The ultrasound transmitter 104 is driven by a first signal generator 116 that passes its energy through a power amplifier 118 thus generating a signal at frequency $f_1$. The ultrasound transmitter 106 is driven by a second signal generator 120 which passes its energy through a power amplifier 122 thus generating a signal at frequency $f_2$. Ideally a small difference in frequency (hertz to kilohertz) between frequency $f_1$ and frequency $f_2$ is created so that a desired beat frequency $\Delta f$ equals $(f_1 - f_2)$ is generated. In response, the target tissue will oscillate at the beat frequency $\Delta f$ at the focal point 112 where the two ultrasound beams 108 and 110 intersect within the desired target area tissue 114. The signal generators 116 and 120 may generate continuous, pulsed, frequency modulated or pulse-delayed waveforms.

While the ultrasound subsystem 100 may be used to vibrate the tissue 114 in the focal point area 112, the RF subsystem 102 may be used to measure the properties of the tissues under analysis and observation in order to provide an image of the tissue structure after signal processing, image reconstruction on the display module 152. The RF subsystem 102 employs a radio frequency source 126 capable of generating radio frequency energy that is amplified by a power amplifier 128. Ideally, a linear power amplifier may be used to amplify the signal sent to a transmit antenna 130 that transmits the radio frequency energy 132 into the tissue 114. The reflected radio frequency energy 134 is collected by a receive antenna 136. In addition to the signal reflecting the signature of the tissues 114, the received signal may include not only the reflected RF carrier of the radio frequency energy 134 but also signal leakage from the transmit antenna, noise interference and signals reflected at the various non-target-related discontinuities within the tissues 114.

Unlike standard radar technology, the extremely short distance between the transmit and receive antenna makes it difficult to turn the transmitter off during the reflected signal reception. The energy reflected from the target tissues 112 represents only a small fraction of the total reflected signal. That fraction is typically between 90 to 120 dB below the carrier signal at the output of the receive antenna thus necessitating the use of advanced cancellation techniques to recover the target-related signal with good fidelity. The primary function of the cancellation module 138 is to produce an output signal equal in amplitude and opposite in phase to the main tone carrier signal from the output of the receive antenna 136. The radio frequency signal 140 received by the receive antenna 136 and a signal with the same amplitude and possessing 180 degrees of phase difference 186 are summed by the RF summer 144. This summed signal 146 represents a signature of the target tissue 114 at the focal point 112. The output of the summer 146 is sent to the RF detector 148. The output 150 of the RF detector 148 is processed and displayed by the signal processing, image reconstruction and display module 152 to produce an overall image of the target 112 within the tissue 114.

The cancellation module 138 functions by taking a bleeded signal 154 from coupler 156 and bleeded signal 184 via coupler 158 that bleeds the received signal 140 to produce a main carrier signal 186 which is equal in amplitude and in 180 degrees phase difference to the received signal 140. An output signal 160 from the RF detector 148 is also sent via path 162 to the cancellation module to ensure that the RF cancellation is adjusted properly so that the RF detector does not see excessive signal amplitudes.

In instances where the receive antenna 136 and the transmit antenna 130 are placed in direct physical contact with tissue 114 under observation and analysis, the antennas may be made from a material that closely matches the dielectric constant of the tissues under analysis in order to improve the efficiency of the radio frequency transmission into the tissues 114 and to minimize the reflection at its surface.

The cancellation module 138 executes a cancellation algorithm by processing the multiple inputs 154, 160 and 184 with input signal 154 coming from hybrid coupler 156 located along the path of the RF source 126, through the power amplifier 128 and the transmit antenna 130; input signal 184 from the hybrid coupler 158 located along path 164 between the receive antenna 136 and the summer 144; and signal output 160 from the RF detector 148 along path 162.

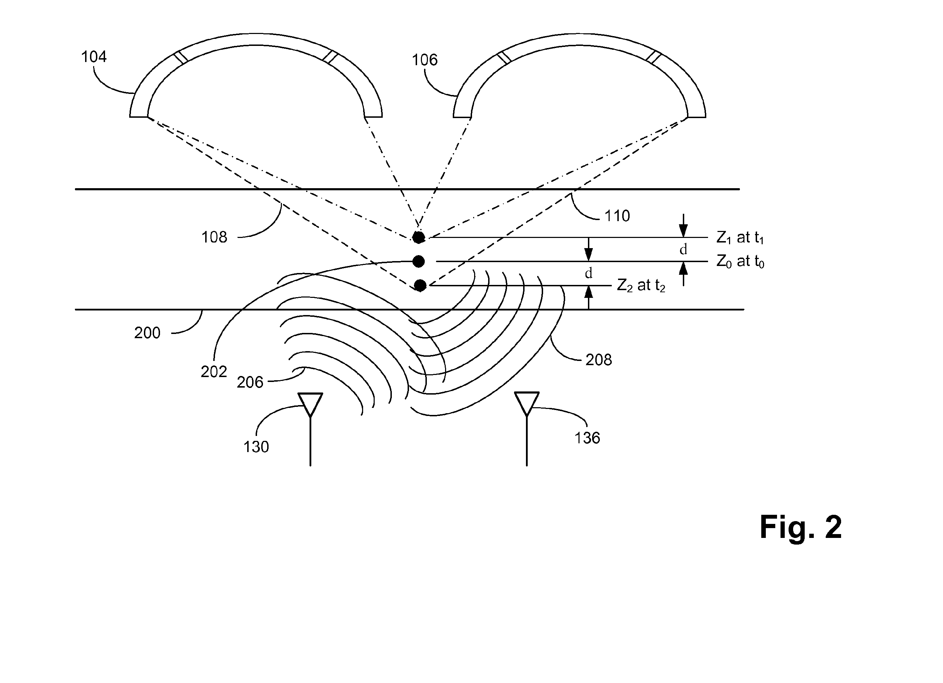

The operation of the dual modality imaging system can be further explained by referring to Figure 2. Figure 2 shows a block diagram of a radio frequency transmit antenna 130 radiating tissue 200 and its inclusion (for example a benign mass, tumor) 202. The receive antenna 136 receiving the radio frequency energy from the vibrating tissues caused by the focused ultrasound beams 108 and 110 emitted from the first and second ultrasound transmitters 104 and 106 respectively with their respective focused beams 108 and 110 intersecting at the object 202 within the tissue 200. The resultant displacement "d" of the target inclusion or object 202 is due to the focused ultrasound waves vibrating the object 202.

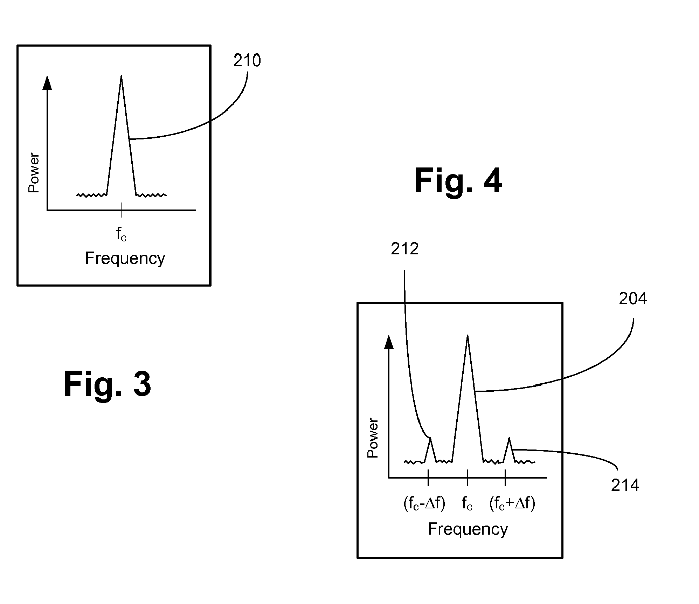

Fig. 3 shows the display of the spectral representation of the radio frequency energy reflected from the tissue 200 and its inclusion 202, including contributions from the direct coupling between the transmit and receive antennas and other reflections, when the ultrasound transmitters 104 and 106 are turned off. Since no ultrasound energy is applied to the target 202, the target is stationary and no sidebands are present. As a result, the spectrum will only show the main unmodulated carrier at a frequency $f_c$ present.

Fig. 4 shows the display of the spectral representation when the two ultrasound transmitters are turned on and are focused on the inclusion 202 which has a dielectric properties that are different from its surrounding tissues. The inclusion 202 will vibrate at a frequency $\Delta f$ which equals the difference between the two ultrasound frequencies ($f_1 - f_2$) resulting in Doppler shift and generation of sidebands 212 and 214 at the frequencies ($f_c - \Delta f$) and ($f_c + \Delta f$) around the carrier $f_c$. The sidebands are a function of the dielectric properties of the inclusion, the difference in the ultrasound frequencies $\Delta f$ and the inclusion displacement d.

Referring back to Figure 2, at time $t_0$, the unexcited inclusion 202 is at rest in location $z_0$ and the radio frequency transmit antenna 136 is transmitting radio frequency energy at a frequency $f_c$ into the tissue 200 that is undergoing analysis. A continuous radio frequency signal may be employed although it is anticipated that other input waveforms and methodologies such as frequency modulation and pulse-delay may be used to reduce clutter signals and improve the probability of tumor detection. Prior to activation of the ultrasound transmitters 104 and 106, radio frequency energy is transmitted by the transmit antenna 130 and reflected by the tissue 200 and the inclusion 202 to the receive antenna 136 and by the internal boundaries separating the various tissue layers 200 as well as from any inclusions or tumors such as target 202. The reflected radio frequency energy is of the same frequency $f_c$ as the transmitted output of the RF source 126. The reflected radio frequency energy appears on a spectral display as a peak 204 at the frequency of the transmitted energy $f_c$ as shown in Figure 3.

At time $t_1$, the ultrasound transmitter 104 transmits its focused ultrasound beam 108 having a frequency $f_1$ into the tissue 200 and the ultrasound transmitter 106 transmits its focused ultrasound beam 110 having a frequency $f_2$ into the target tissue 200. In Figure 2, the ultrasound transmitters are confocal; however, other transmitter configurations including an array of ultrasound sources or confocal ultrasound arrays may be used. The lenses of the ultrasound transmitters 104 and 106 are designed to create focused ultrasound beams such that their focal points are coincident at the target inclusion 202. The ultrasound frequencies $f_1$ and $f_2$ are typically high frequencies with a small differential or beat frequency $\Delta f = (f_1 - f2)$. The high frequencies of the input ultrasound waves 108 and 110 provide superior resolution and focus capability, but poor tissue displacement force. As the first and second high-frequency ultrasound waves propagate and interact, the non-linearity introduced by the inclusion (or tumor) produce a series of harmonic waves. One resultant harmonic is a low-frequency wave at the beat frequency $\Delta f = (f_1 - f_2)$ resulting from the constructive and destructive interference of the high-frequency components of the input waves. This low-frequency harmonic component produces a force that excites and displaces the target tissue and the inclusion 202. Due to the non-linear density and elastic properties of tissues and other structures such as tumors, lesions, ablations, etc., the target structure 202 can be displaced and thus detected. Expressed mathematically:

$$Source\ 1 = \cos(2\pi f_1 t) = cos(\omega_1 t)$$

$$Source\ 2 = \cos(2\pi f_2 t) = cos(\omega_2 t)$$

where $\omega = 2\pi f$ is the angular frequency and t = time.

Due to high power at the intersection point of the ultrasound beams, non-linear effects of the tissue become pronounced and the mixing of the two ultrasound signals becomes:

$$Resultant = a_1[cos(\omega_1 t) + cos(\omega_2 t)] + a_2[cos(\omega_1 t) + cos(\omega_2 t)]^2 + \cdots$$

or

$$Resultant = a_1 cos(\omega_1 t) + a_1\ cos(\omega_2 t) + a_2 cos^2(\omega_1 t) + a_2 cos^2(\omega_2 t)$$
$$+ 2a_2 cos(\omega_1 t)cos(\omega_2 t) + \cdots$$

So that

$$Resultant = a_1 cos(\omega_1 t) + a_1\ cos(\omega_2 t) + a_2[0.5 cos(2\omega_1 t) + 0.5]$$
$$+ a_2[0.5 cos(2\omega_2 t) + 0.5]$$
$$+ a_2\big[cos\big((\omega_1 + \omega_2)t\big) + cos\big((\omega_1 - \omega_2)t\big)\big] + \cdots$$

where the "$a_i$" coefficients are dependent upon the non-linearity of the tissue.

The resultant displacement d of the tissue is given by the equation:

$$d = \left(\frac{1}{2\pi f}\right)\sqrt{2FZ}$$

where F = energy flux (i.e., power per area), Z = tissue acoustic impedance, typically $\sim$ 1.5e6 kg/m$^2$/s, and $f$ = acoustic frequency, in this case is the beat frequency $\Delta f = (f_1\text{-}f_2)$.

Since $\omega_1$ and $\omega_2$ are high frequencies to achieve good resolution, then terms with twice the frequency (cos ($2\omega_1$), cos ($2\omega_2$) and cos ($\omega_1\text{+}\omega_2$)) will be of high frequency and their effect on the motion will be limited. On the other hand, if $\omega_1$ and $\omega_2$ are selected to be close to each other such that ($\omega_1\text{-}\omega_2$) would be very small (i.e., in order of hundreds to thousands of Hertz), then the term cos (($\omega_1\text{-}\omega_2$)t) will lead to the desired large displacement. It should be noted that the displacement d is inversely proportional to the ultrasound beat frequency.

At time $t_2$, the low-frequency ultrasound component (of frequency $\Delta f = f_1\text{-}f_2$) impacts the inclusion or tumor 202 and displaces the tumor 202 to location $z_2$. As the low-frequency ultrasound wave passes the inclusion 202, the inclusion oscillates between location $z_2$ and $z_1$ before coming to rest again at essentially the initial location $z_0$. The ultrasound waves 108 and 110 travel at a significantly lower rate of speed than the radio frequency signals 206 and 208. The oscillation of the inclusion 202 between position $z_1$ and position $z_2$ results in a Doppler effect which in turn results in a shift in the frequency of the reflected radio frequency signal. The graph in Figure 3 illustrates the reflected radio frequency spectrum when the ultrasound energy is off such as the case when the inclusion 202 is at rest in position $z_0$. Here, the spectrum comprises the background and a peak 210 at the radio frequency carrier frequency $f_c$. Along with the reflected carrier peak and the background noise, the graph in Figure 4 displays the reflected radio frequency spectrum when the ultrasound energy is on and the frequency sidebands 212 and 214 are present due to the inclusion cyclic displacement. The presence of these sidebands indicates the presence of tissue discontinuities, inclusions such as the presence of a tumor 202 or where the tissue has different dielectric properties than the adjacent tissues. The sidebands 212 and 214 are present only when the ultrasound beams are present and intercepting the target 202. The RF sideband energy reflected from the target is due to the difference in dielectric properties of cancerous lesions compared to the background tissue.

The power level of the sidebands 212 and 214 is determined through displacement analysis. If a signal is reflected off of a target whose range is changing with time according to $r(t) = r_0 + \Delta r(t)$, the received signal can be written as:

$$s(t) = cos\left[\omega_c t + \frac{2\pi\Delta r(t)}{\lambda} + \varphi_0\right]$$

where $\omega_c$ is the carrier angular frequency and $\varphi_0$ is the phase

For a small-amplitude oscillation of a target with a displacement d and a modulation frequency $f_m$, the range is given by:

$$\Delta r(t) = d sin(\omega_m t)$$

and thus the signal becomes

$$s(t) = cos\left[\omega_c t + \frac{2\pi d sin(\omega_m t)}{\lambda} + \varphi_0\right]$$

For d $<<$ $\lambda$ this expression is simply the narrowband FM situation:

$$f(t) = \cos\left[\omega_c t + \frac{d}{\lambda} sin(\omega_m t)\right]$$

or

$$f(t) = cos(\omega_c t)cos\left(\frac{d}{\lambda}sin(\omega_m t)\right) - sin(\omega_c t)sin\left(\frac{d}{\lambda}sin(\omega_m t)\right)$$

Since $d/\lambda \ll 1$,

$$f(t) \cong cos(\omega_c t) - \left(\frac{d}{2\lambda}\right)\left[cos\left((\omega_c - \omega_m)t\right) - cos\left((\omega_c + \omega_m)t\right)\right]$$

Each sideband is smaller than the carrier by:

$$P_{sideband} = 10log\left(\frac{d^2}{4\lambda^2}\right) = 20log\left(\frac{\pi f_c d}{c}\right) \ dBc$$

Radio frequency sensitivity is determined by the equation:

$$Sensitivity = NF + kT + 10\log(BW) + SNR - 10log(Average)$$

where:

NF: The receiver input referred noise figure (Typically 3-5dB)
kT: Thermal noise power density (-174dBm/Hz)
BW: Receiver noise bandwidth in Hz (typically1-2 MHz)
SNR: Required detector SNR in dB (20dB)
Average: Coherently collected samples over sample time

If sensitivity is not sufficient, and to give system sensitivity a boost, a continuous wave may be employed such that:

$$Sensitivity = NF + kT + 10\log(BW) + SNR - 10log(Average)$$
$$- 10\log(gain)$$

where the higher gain is achieved due to applying continuous wave power.
The advantages of using radar technology for the imaging of tissue structures such as tumors, lesions, inclusions or ablations through detection of the reflected sidebands resulting from the low frequency oscillation of the embedded inclusion is that excellent discrimination can be achieved as a result of the differences in dielectric properties of cancerous versus non-cancerous tissues. The reflected radio frequency signal is a function of the dielectric properties of the tissue and also the dielectric properties of any inclusion or tumor buried in that tissue. Imaging based on dielectric properties is superior to the use of the reflected acoustic energy which is a function of the tissue mechanical properties. For comparison purposes the relative energy reflected by an inclusion can be estimated for cases where elastic properties provide the means of contrast with the surrounding tissue and also for the case where dielectric properties provide the contrast. The advantages of using dielectric properties versus elastic properties for contrast can be estimated using plane wave analysis. For plane waves incident on a boundary between two media the reflected energy is related to the incident energy by $\rho_R^2$. Where $\rho_R$ is defined by the equation:

$$\rho_R = \left(\frac{Z_2 - Z_1}{Z_2 + Z_1}\right)$$

and $Z_1$ is the characteristic impedance of medium 1 and $Z_2$ is the characteristic impedance of medium 2.

With regard to the elastic properties, the acoustic impedance $Z_a$ is defined as

$$Z_a = \sqrt{\rho_d \kappa}$$

where $\rho_d$ is the density and $\kappa$ is the adiabatic elastic modulus. Since the elastic properties vary little from non-cancerous to cancerous tissues, the elastic constant in medium 2 ($\kappa + \Delta\kappa$) can be related to that in medium 1 ($\kappa$) under the assumption that $\Delta\kappa << \kappa$.

With this assumption the acoustic reflection coefficient is

$$\rho_{Ra} \cong \frac{\Delta\kappa}{4\kappa}$$

If $\Delta\kappa/\kappa$ has a typical value in the range of 1%, then $\rho_{Ra} \cong 0.0025$.

Likewise, the electrical characteristic impedance $Z_e$ for a plane wave is:

$$Z_e = \sqrt{\frac{\mu_0}{\varepsilon_0}} \sqrt{\frac{\mu_r}{\varepsilon_r}}$$

where the subscript 0 refers to the absolute permeability/susceptibility and the subscript r refers to the relative value. For cancerous breast tissue, for example, the magnetic permeability is unchanged but the value of the relative dielectric constant increases by a factor of 4. In this case:

$$\rho_e = \frac{1}{3}$$

The higher value of reflection coefficient in case of dielectric contrast will give much more reflected energy. For this simple plane wave analysis the ratio is approximately 42.5dB. It should be recognized that there will be multiple reflecting boundaries as well as attenuation and multiple pathways for the energy to transit from the transmitter to the receiver. Nevertheless, the significantly higher contrast the electrical permittivity provides cannot be overlooked and is likely to always be a significant improvement over the contrast provided by use of elastic properties.

It will be recognized by those skilled in the art that phenomena other than the physical displacement of the target tissue can give rise to the appearance of sidebands. For example, if the sound pressure is sufficient at the target site, the target itself may undergo periodic physical deformation which may give rise to an incremental periodic variation of the relative dielectric constant. Such a variation would also produce small sidebands around the carrier signal that is reflected to the receive antenna.

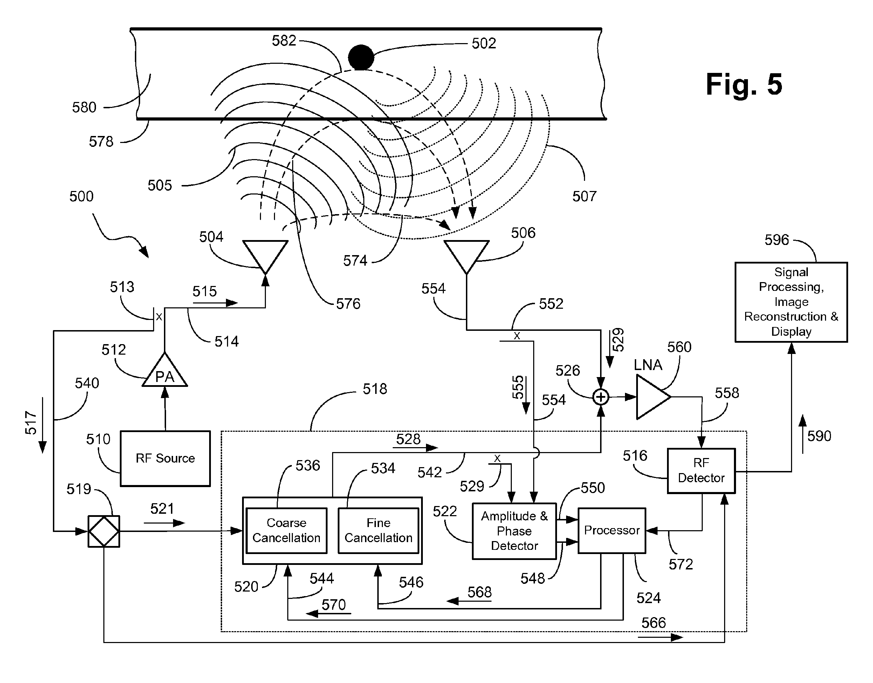

Figure 5 describes the operation of the radio frequency subsystem 500. The subsystem operates as a high dynamic range radar with extremely short range which is capable of extracting the sidebands generated as a result of the Doppler shift generated by the low frequency acoustic vibrations generated by the inclusion 502 when the inclusion is subjected to focused ultrasound waves. The need for 90 to 120 dB dynamic range is dictated by the need to keep the transmitted signal on during the reflected signal detection. The first antenna 504 and second antenna 506 may be RF antennas operating in the microwave region of the electromagnetic spectrum. Antennas 504 and 506 may also be transmitter 504 and receiver 506 antennas, transceiver antennas or an antenna with a diplexer used to separate the transmitted and reflected radio frequency antennas.

Figure 5 is a block diagram of an example of an implementation of a RF subsystem 500. The subsystem 500 may include a transmit antenna 504 capable of transmitting RF signals 505, a coupler 513, a receive antenna 506 capable of receiving reflected RF signals 507, an RF source 510 which couples the radio frequency energy to the transmit antenna 504 through the power amplifier 512 via path 514, an RF detector 516, cancellation module 518 which includes an amplitude and phase shift module 520, an amplitude and phase detector module 522, and a processor 524, and a combiner 526. The amplitude and phase shift module 520, under control of the processor 524, generates a signal 528 which is equal to or close in magnitude and opposite in phase to the main carrier tone of signal 529 at the output of the receive antenna 506 thus cancelling the received signal at the carrier frequency $f_c$.

The RF source 510 may be an oscillator, a temperature controlled oscillator or any other type of frequency generating device. The radio frequency energy generated by the RF source 510 is passed to an amplifier 512 and then on to the transmitting antenna 504 along path 514. A coupler 513 is located on signal path 514 bleeds the radio frequency energy signal 517 along path 540 to a signal splitter/attenuator 519. From the splitter/attenuator 519, part of the radio frequency energy 521 is sent to the amplitude and phase shift module 520 while the other part is of the radio frequency energy 566 is sent to the RF detector 516.

The amplitude and phase shift module 520 further comprises fine and coarse cancellation modules 534 and 536. The amplitude and phase shift module 520 receives input signals from the signal splitter/attenuator 519 as well as from the processor 524. The output signal 528 of the amplitude and phase shift module 520 is passed to the combiner 526 via signal path 542. The amplitude and phase shift module 520 receives signals 568 and 570 from the processor 524 via signal paths 544 and 546 respectively thus feeding digital signals into the coarse cancellation module 536 and the fine tune cancellation module 534. The processor 524 is in signal communication with the amplitude and phase detector module 522 via signal paths 548 and 550 (one path 548 conveying amplitude and/or phase information regarding path 528 while the other path 550 conveys the amplitude and/or phase information regarding path 554).

The receive antenna 506 is in signal communication with combiner 526 via path 554 and the amplitude and phase detector 522 via the coupler 552 via signal path 554. The combiner 526 output connects with the low noise amplifier 560. The output from low noise amplifier 560 is sent via path 558 to the RF detector 516. The RF detector 516 outputs a low frequency signal 590 to the signal processing, image reconstruction and display 596.

It will be clear to those skilled in the art that the imaging output can be used to drive a display. The result will be a stand-alone imaging system with a self-contained imaging subsystem. Similarly the imaging output can be used in conjunction with a system based on another imaging modality. In this way the images can be overlaid, combined or otherwise utilized to improve diagnostic results. The image output from this invention can be in digital or analog signal format and may be presented to the user as an electric display, film, or other media consistent with medical industry standard practice.

The RF detector 516 may be a circuit, component, module, and/or device includes at least one mixer (not shown) and is capable of receiving radio frequency signals from the output of the LNA 560 and a reference RF signal 566 from the splitter/attenuator 519. The output of the RF detector 516 is sent to the processor 524 via path 572. The RF detector 516 could incorporate digital and or signal processing capabilities to further enhance the target image of the tissue, improve and calibrate the system noise performance or condition the received signal to further enhance the input to the processor 524.

The amplitude and phase detector module 522 may be a circuit, component, module, and/or device. The amplitude and phase detector module 522 is capable of receiving (1) the received radio frequency amplitude and phase of signal 555 along path 554 from coupler 552; and (2) the output 528 from coupler 529 which is output of the amplitude and phase shift module 520. The amplitude and phase detector module 522 may include one or more power detector sensor circuits and/or phase detector sensor circuits.

In an example operation of the amplitude and phase detector 522, the amplitude and phase detector module 522 produces a first signal along path 548 in response to detecting the amplitude and/or phase of the output signal 528 and a second signal along path 550 in response to detecting the amplitude and/or phase of the received signal along path 554. The amplitude and phase detector module signals sent along paths 548 and 550 respectively may be sent to the processor 524 as analog signals or digital data containing information regarding the amplitude and/or phase of the received signal on path 554 and the output of the of the amplitude phase shift module signal along path 542. If analog data is sent, an analog to digital converter is needed before the signal is passed to the processor 524.

The processor 524 may be a circuit, component, module, and/or device capable of receiving the amplitude and phase detector module signals along paths 548 and 550, and in response may generate amplitude phase shift module control signals 568 and 570 that would control the fine and coarse cancellation modules of the amplitude and phase shift module 520. The processor 524 may be capable of determining how to modify the amplitude and/or phase of the transmit signal 528 with the amplitude phase shift module 522 in order to increase resolution and dynamic range of the receiver output signal 564 based on the measured power and/or phase information data provided by the amplitude and phase detector module signals sent along paths 548 and 550. The processor 524 may be, for example, a central processing unit ("CPU"), microprocessor, microcontroller, controller, digital signal processor ("DSP"), reduced instruction set processor ("RISC processor"), application specific integrated circuit ("ASIC"), or other similar types of devices.

The cancellation module 518 acts to cancel the main carrier tone as well as any direct coupling leakage signals 574 that are passed directly from the transmit antenna 504 to the receive antenna 506. Also cancelled are any leakage signals 576 from the transmit antenna 504 to the receive antenna 506 that may occur resulting from the radio frequency energy reflections at the near surface 578 of the tissue 580 under analysis and test, and main tone carrier signals 582 resulting from the radio frequency energy impinging the inclusion under analysis and test. Signals 582 will generate sidebands due to the Doppler shift resulting from the vibration of the inclusion 502. Thus, the cancellation system module 518 is designed to cancel the main tones from signal 582, plus signals 574 and 576, leaving only the sidebands generated from the signal 582.

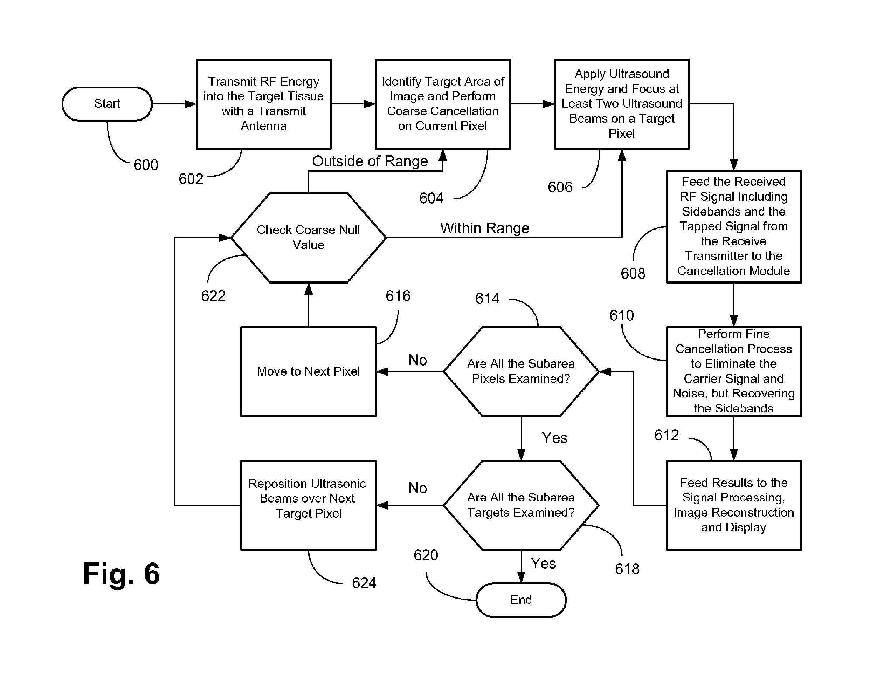

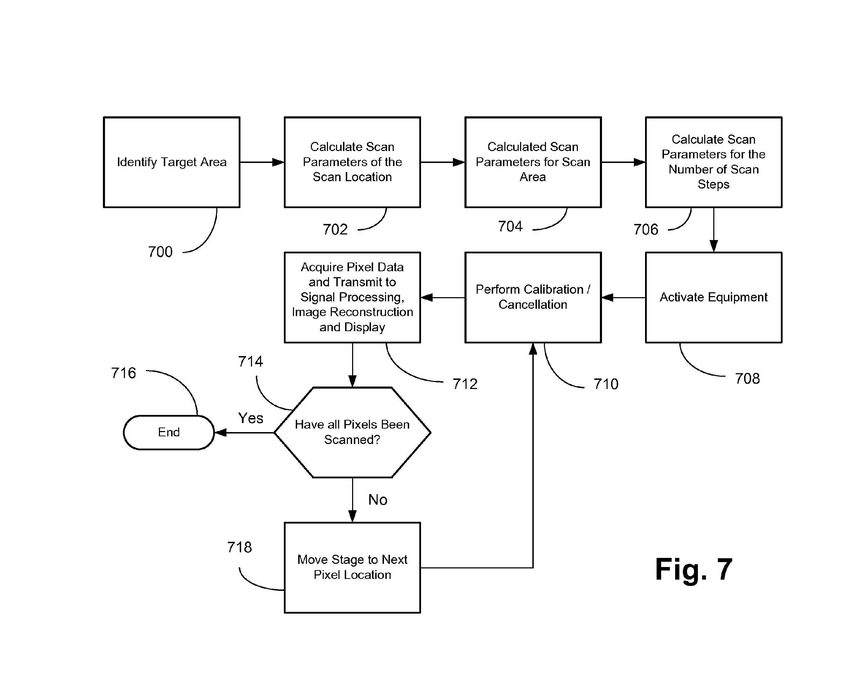

Figure 6 is a flow chart illustrating the operation of the dual modality imaging system. This flow chart details the flow of the processes to image the target tissue using a scanning approach. Other embodiments may use other implementations different from scanning such as hand-held probes or other methods incorporating the dual modality. However, the implementation may vary depending on the nature of the target tissue to be imaged such as the case with breast cancer imaging, or the imaging of internal organs such a liver, pancreas or cardiac imaging as well as image-guided surgeries. The process outlined by the flow chart in Fig. 6 assumes the boundaries of the target tissue to be imaged have been identified, and have been divided into scannable subareas, a step that will be described in Figure 7.

Referring to Figure 6, the process starts 600 upon the application of radio frequency energy to the target tissue under test 602. The RF source is tuned to the desired RF frequency and is applied to the target tissue with its inclusion. While the ultrasound power is off, the reflected radio frequency energy is received by the receive antenna 506 and the received signals are passed through the cancellation module, along with the reference signal from the RF source so that the cancellation module calibration may be performed. A coarse cancellation is performed 604 for the current pixel. The ultrasound power is turned on during process 606 and the two ultrasound transmitters 104 and 106 are tuned on and tuned to the desired frequencies $f_1$ and $f_2$ to ensure that their respective beams 108 and 110 are focused with their focal points intersecting at the inclusion 502 of the target tissue 580 which will cause the inclusion 502 to vibrate at the beat frequency $(f_1 - f_2)$.

Next in step 608 the reflected RF signals 507 including the Doppler sidebands resulting from effects of the ultrasound beat frequency vibration of the tissue along with the reference signal 566 is fed to the cancellation module 520. During process 610 the fine tune cancellation process is performed and the pixel data is fed to the image processor 524.

The results of the fine tune cancellation process 610 are then fed to the signal processing, image reconstruction and display 612. A test is then performed inquiring as to whether all the subarea pixels have been examined 614. If the response is no, the process moves to the next pixel 616. However, if the response is yes, a further test is inquired as to whether all the subarea targets have been examined 618. If the answer to the inquiry is yes, the process ends 620.

The movement from one pixel to the next pixel may be accomplished by serial scanning a small focal point over a larger scan area. However, other methodologies may be employed. The system may scan by a mechanical mechanism moving the focal point pixel by pixel or electronic means may be employed so that the focused ultrasound beams are steered electronically. Also, the focal point of the at least two ultrasound sources are repositioned to a greater or lesser depth within the target tissue to enable acquisition of images at various depth planes.

Returning to step 614, if the answer to the inquiry to whether all the subarea pixels have been examined 614, is no, the process moves to the next pixel 616. The process then inquires as to whether the coarse null value has been reached 622. If the response is that the process is within a predetermined range, then the process repositions the ultrasonic beams over the next target pixel and invokes process 606. If the response to step 622 is outside of the predetermined range, then the system invokes process 604 and performs the coarse cancellation on the current pixel.

Step 614 checks if all pixels in the current subareas has been examined. If more pixels remains in the current subarea the ultrasound beams are moved to the next pixel 616, otherwise step 618 checks if all the subarea targets have been examined, which will decide if the imaging process has been completed or if step 624 will be invoked which in turn may move the stage to the next subarea or reposition the ultrasound beams to focus on the next subarea. Referring to step 616, the use of stage movement or the refocusing of the ultrasound beam is a function of the hardware implementation and the type of ultrasound sources being used. After the next subarea has been defined, step 622 will be invoked; if the coarse cancellation null is still within the predetermined range, then there is no need to perform the exhaustive coarse cancellation process which in turn will speed up the imaging process considerably, otherwise the extensive coarse search 604 will be invoked. The predetermined range for the cancelled signal may be a power setting such as 20-40 dB; one degree of phase difference; or some other user specified criteria that approximates the amplitude and phase of the received signal so that the main carrier tone can be cancelled.

It will be understood by those skilled in the art that the scannable subareas need not necessarily be only laterally disposed with respect to one another so that all scanning occurs in one vertical plane. The use of at least two focused ultrasonic beams allows the beams to be focused to different depth planes within the target area so that, if necessary, a three dimensional image of the target area can be made.

Figure 7 is a flow chart outlining the scanning procedure. It should be noted that, instead of a scanner there are other methodologies that may be utilized such as employing a hand-held probe similar to probes used with current ultrasound imaging systems, non-invasive probes used in Trans-Esophageal Echocardiograms or in semi-invasive probes used for cardiac imaging. The procedure starts by identifying the target area 700. The purpose of this process is to identify the outline of the target area to be imaged. This can be done by using a lower resolution image of the target which can be a B-mode ultrasound image or by relying on an image from another imaging modality. If ultrasound B-mode is used to identify the image target area, either one of the ultrasound transmitters 104 or 106 may be replaced by an ultrasound transceiver. In step 702 the target area is divided into the appropriate number of subareas. Step 704 calculates the scan parameters for the subareas such as the number of pixels per subarea, the pixel size and the algorithm for sequencing the subareas and the pixels. It also defines the first pixel to be scanned. The process of defining the subareas may be calculated based on the number of scan steps 706 or may depend on the desired quality of the target image and the finding of the low resolution image resulting from step 700.

The equipment is activated during step 708 and the examination of the first pixel of the first subarea is started. During step 708, the amplitude and phase shift module 520 performs the cancellation module calibration by running an exhaustive coarse calibration algorithm and fine tune cancellation on the first pixel. The imaging system acquires the current pixel data 712 and sends it to the signal processing and image reconstruction module 596.

The system then acquires the data associated with the sidebands and sends it to the signal processing, image reconstruction and display 712. The next step checks to determine whether all the pixels have been scanned 714. If the response to the inquiry is yes, the process ends 716. If the response to the inquiry is no, the process moves the ultrasound beams to focus on the next pixel location 718 and the system returns to step 712 repeating the sequence of steps.

In some cases the exact depth of the target in the tissue may be unknown. For example, mammography provides a two dimensional image of any anomaly. In that case, it may be appropriate to scan laterally to correlate the shape with the mammography image and by refocusing the ultrasound beams at different depths to measure of the vertical extent of the anomaly and to form a three dimensional image of the target.

While various embodiments of the invention have been described, it will be apparent to those of ordinary skill in the art that many more embodiments and implementations are possible that are within the scope of this invention.

**Claims**

1.    A system for imaging a target tissue, comprising:

a radio frequency source capable of generating radio frequency energy and transmitting the radio frequency energy through the target tissue (114);
at least two ultrasound sources (104 and 106) with a difference in frequency capable of generating focused ultrasound waves (108 and 110), transmitting the focused ultrasound waves through the target tissue (114) exciting the target tissue thereby generating sidebands (212 and 214) around a reflected radio frequency carrier (204); and
a radio frequency detector (148) capable of receiving reflected radio frequency energy bouncing off the excited target tissue (114);
**characterized in that** the system for imaging a target tissue further comprises:
a cancellation module capable (138) of analyzing the reflected radio frequency energy and cancelling out main carrier tone reflections (204) so that the sidebands remain (212 and 214); and
a digital signal processor (152) capable of generating an output that represents the image of the target tissue.

2.    The system for imaging the target tissue of claim 1, further comprising a programmable controller (152) determines the difference in ultrasound frequency between the focused ultrasound waves.

3.    The system for imaging the target tissue of claim 1, where the focused ultrasound waves are focused on the target tissue (114) resulting in a frequency shift when the radio frequency energy is reflected from the target tissue (502).

4.    The system for imaging the target tissue of claim 1, where the digital signal processor (152) is capable of producing an image of the target tissue from analysis of the sidebands (212 and 214) resulting from the difference in dielectric properties of the target tissue (502) and surrounding tissues (580).

5.    The system for imaging the target tissue of claim 1, where the radio frequency energy supplied by the radio frequency source (126) is split into first (132) and second parts (154), where the first part is directed to the target tissue (114) and the second part (154) is used to cancel out unmodulated parts of the radio frequency energy in the first part that is collected by a radio frequency detector (148).

6. The system for imaging the target tissue of claim 1, where a cancellation module is used to generate a signal equal in amplitude and opposite in phase to the main carrier tone reflections (204) captured by the radio frequency detector (148).

7. The system for imaging the target tissue of claim 1, where a coarse cancellation module (536) is used to adjust amplitude of the radio frequency energy main carrier tone reflections so as to generate a signal that acts to cancel amplitude of the main carrier tone reflections (204) of the reflected radio frequency energy (134).

8. The system for imaging the target tissue of claim 1, where a coarse cancellation module (536) is used to adjust the phase of the radio frequency energy main carrier tone reflections so as to generate a signal that acts to cancel the main carrier tone reflections (204) of the reflected radio frequency energy (134).

9. The system for imaging the target tissue of claim 1, where a coarse cancellation module (536) is used to adjust amplitude and phase of the radio frequency energy main carrier tone reflections so as to generate a coarse cancellation output that acts to cancel the main carrier tone reflections (204) of the reflected radio frequency energy (134).

10. The system for imaging the target tissue of claim 1, where a fine tune cancellation module is used in conjunction with the coarse cancellation module (536) to generate the same amplitude of the radio frequency energy with 180 degrees of phase difference as the main carrier tone reflections (204) of the reflected radio frequency energy (134).

11. The system for imaging the target tissue of claim 7, where the coarse cancellation module (536) generates a coarse output cancellation output whose amplitude resembles the amplitude of the main carrier tone reflections (204) of the reflected radio frequency energy (134).

12. The system for imaging the target tissue of claim 9, where the coarse cancellation output is further refined by a fine tune cancellation module (534) capable of generating a fine tune cancellation output whose amplitude resembles the main carrier tone reflections amplitude of the reflected radio frequency energy and its phase is approximately 180 degrees out of phase with the main carrier tone reflections (204) of the reflected radio frequency energy (134).

13. The system for imaging the target tissue of claim 9, where the coarse cancellation output is further refined by a fine tune cancellation module (534) capable of generating a fine tune cancellation output whose amplitude resembles the main carrier tone reflections amplitude of the reflected received radio frequency energy and is based on a predetermined criteria with respect to the main carrier tone reflections (204) of the reflected radio frequency energy (134).

14. The system for imaging the target tissue of claim 9, where the coarse cancellation output is further refined by a fine tune cancellation module (534) capable of generating a fine tune cancellation output whose phase resembles the main carrier tone reflections phase of the reflected received radio frequency energy and based on a predetermined criteria with respect to the main carrier tone reflections (204) of the reflected radio frequency energy (134).

15. The system for imaging the target tissue of claim 7, where the coarse cancellation module (536) is bypassed if the coarse cancellation module output is within a predetermined range.

16. The system for imaging the target tissue of claim 1, where the radio frequency energy is transmitted and received by an antenna (136) having a diplexer used to separate the transmitted and reflected radio frequency energy (134).

17. The system for imaging the target tissue of claim 1, where the at least two ultrasound sources are confocal (104 and 106).

18. The system for imaging the target tissue of claim 1, further comprising a detector that is capable of analyzing the reflected radio frequency energy (134) and cancelling direct couplings (574) of radio frequency energy between a transmit and a receive antenna (136).

Fig. 1

EP 2 692 289 A1

Fig. 2

EP 2 692 289 A1

Fig. 4

Fig. 3

Fig. 5

**Fig. 6**

Start — 600

Transmit RF Energy into the Target Tissue with a Transmit Antenna — 602

Identify Target Area of Image and Perform Coarse Cancellation on Current Pixel — 604

Apply Ultrasound Energy and Focus at Least Two Ultrasound Beams on a Target Pixel — 606

Outside of Range

Check Coarse Null Value — 622

Within Range

Feed the Received RF Signal Including Sidebands and the Tapped Signal from the Receive Transmitter to the Cancellation Module — 608

Perform Fine Cancellation Process to Eliminate the Carrier Signal and Noise, but Recovering the Sidebands — 610

Move to Next Pixel — 616

No

Are All the Subarea Pixels Examined? — 614

Yes

Feed Results to the Signal Processing, Image Reconstruction and Display — 612

Reposition Ultrasonic Beams over Next Target Pixel — 624

No

Are All the Subarea Targets Examined? — 618

Yes — 620

End

EP 2 692 289 A1

EP 2 692 289 A1

```
┌──────────────┐     ┌──────────────┐     ┌──────────────┐     ┌──────────────┐
│              │     │ Calculate Scan│     │              │     │ Calculate Scan│
│Identify Target│────▶│Parameters of the│──▶│Calculated Scan│──▶│Parameters for the│
│    Area      │     │ Scan Location │     │Parameters for Scan│ │ Number of Scan│
│              │     │              │     │    Area      │     │    Steps     │
└──────────────┘     └──────────────┘     └──────────────┘     └──────────────┘
       702                704                  706
       700
```

Identify Target Area

Calculate Scan Parameters of the Scan Location — 702

Calculated Scan Parameters for Scan Area — 704

Calculate Scan Parameters for the Number of Scan Steps — 706

700

Acquire Pixel Data and Transmit to Signal Processing, Image Reconstruction and Display — 712

Perform Calibration / Cancellation — 710

Activate Equipment — 708

714

Have all Pixels Been Scanned? — 716

Yes → End

No

Move Stage to Next Pixel Location — 718

**Fig. 7**

Europäisches
Patentamt

European
Patent Office

Office européen
des brevets

# EUROPEAN SEARCH REPORT

Application Number

EP 13 17 8301

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | US 2009/281422 A1 (SALAMA KHALED N [US] ET AL) 12 November 2009 (2009-11-12) * the whole document * | 1 | INV. A61B5/00 A61B5/05 A61B8/08 |
| A | US 2005/004466 A1 (HYNYNEN KULLVERO H [US] ET AL HYNYNEN KULLERVO H [US] ET AL) 6 January 2005 (2005-01-06) * paragraphs [0026] - [0031]; figures * | 1 | |
| A,D | US 5 099 848 A (PARKER KEVIN J [US] ET AL) 31 March 1992 (1992-03-31) * the whole document * | 1 | |
| A | BUERKLE A ET AL: "Analysis of Acousto-Electromagnetic Wave Interaction Using the Finite-Difference Time-Domain Method", IEEE TRANSACTIONS ON ANTENNAS AND PROPAGATION, IEEE SERVICE CENTER, PISCATAWAY, NJ, US, vol. 55, no. 8, 1 August 2008 (2008-08-01) , pages 2191-2199, XP011232457, ISSN: 0018-926X * abstract; sections I, II and VI; last paragraph of the right-hand col. on p. 2198 * | 1 | TECHNICAL FIELDS SEARCHED (IPC) A61B |

-/--

The present search report has been drawn up for all claims

| Place of search | Date of completion of the search | Examiner |
|---|---|---|
| Munich | 9 December 2013 | Küster, Gunilla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 13 17 8301

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PENG JIANG ET AL: "A new tissue harmonic imaging scheme with better fundamental frequency cancellation and higher signal-to-noise ratio", 1999 IEEE ULTRASONICS SYMPOSIUM. PROCEEDINGS. INTERNATIONAL SYMPOSIUM (CAT. NO.99CH37027), vol. 2, 1 January 1998 (1998-01-01), pages 1589-1594, XP055092319, ISSN: 1051-0117, DOI: 10.1109/ULTSYM.1998.765248 ISBN: 978-0-78-035722-8 * abstract; section II * | 1 | |

TECHNICAL FIELDS
SEARCHED (IPC)

The present search report has been drawn up for all claims

| Place of search | Date of completion of the search | Examiner |
|---|---|---|
| Munich | 9 December 2013 | Küster, Gunilla |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 13 17 8301

This annex lists the patent family members relating to the patent documents cited in the above-mentioned European search report.
The members are as contained in the European Patent Office EDP file on
The European Patent Office is in no way liable for these particulars which are merely given for the purpose of information.

09-12-2013

| Patent document cited in search report | | Publication date | Patent family member(s) | | Publication date |
|---|---|---|---|---|---|
| US 2009281422 | A1 | 12-11-2009 | NONE | | |
| US 2005004466 | A1 | 06-01-2005 | US | 2005004466 A1 | 06-01-2005 |
| | | | WO | 2005002447 A1 | 13-01-2005 |
| US 5099848 | A | 31-03-1992 | AU | 8925991 A | 26-05-1992 |
| | | | CA | 2095440 A1 | 03-05-1992 |
| | | | EP | 0556257 A1 | 25-08-1993 |
| | | | JP | H06504453 A | 26-05-1994 |
| | | | US | 5099848 A | 31-03-1992 |
| | | | WO | 9207513 A1 | 14-05-1992 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20070276240 A1, Rosner
- US 5099848 A, Parker
- US 6448788 B
- US 6876879 B
- US 7266407 B, Li
- US 20090281422 A1, Salama